# EUROPÄISCHE PATENTSCHRIFT

(11) **EP 2 336 114 B1**
(45) Veröffentlichungstag und Bekanntmachung des Hinweises auf die Patenterteilung: **17.04.2013**
(21) Anmeldenummer: 11001114.5
(22) Anmeldetag: 21.11.2008
(51) Int. Cl.: C07D 233/32, C07C 229/00

(54) **VERFAHREN ZUR HERSTELLUNG VON L-3,4 DIHYDROXY-6-[18F]FLUOR-PHENYLALANIN UND 2-[18F]FLUOR-L-TYROSIN UND DEREN ALPHA-METHYLIERTEN DERIVATEN AUS EINEM VORLÄUFER**
PROCESS FOR THE SYNTHESIS OF L-3,4 DIHYDROXY-6-[18F]FLUORO-PHENYLALANINE AND 2-[18F]FLUOR-L-TYROSINE AND THEIR ALPHA-METHYLATED DERIVATIVES STARTING FROM A PRECURSOR
PROCÉDÉ DE PRODUCTION DE L-3, 4 DIHYDROXY-6-[18F] FLUORO-PHÉNYLALANINE ET 2 - [18F] FLUOR-L-TYROSINE ET LEURS DÉRIVÉS ALPHA-MÉTHYLÉS PARTIR D'UN PRÉCURSEUR

(30) Priorität: 07.12.2007 DE 102007059313
(43) Veröffentlichungstag der Anmeldung: 22.06.2011
(62) Teilanmeldung aus: 08857332.4
(73) Patentinhaber: Forschungszentrum Jülich GmbH, 52425 Jülich (DE)
(72) Erfinder: Wagner, Franziska, 85599 Hergolding (DE); Ermert, Johannes, 51109 Köln (DE); Coenen, Heinrich Hubert, 41516 Grevenbroich (DE)

(56) Entgegenhaltungen:
- KURODA C ET AL: "Synthesis of a chiral precursor for no-carrier-added (NCA) PET tracer 6- [<18>F]fluoro-L-dopa based on regio- and enantioselective alkylation of 2,4- bis(chloromethyl)-5-iodoanisole", BULLETIN OF THE CHEMICAL SOCIETY OF JAPAN 200002 JP LNKD- DOI:10.1246/BCSJ.73.417, Bd. 73, Nr. 2, Februar 2000 (2000-02), Seiten 417-422, XP002634866, ISSN: 0009-2673
- SHEN ET AL: "Decarbonylation of multi-substituted [<18>F]benzaldehydes for modelling syntheses of <18>F-labelled aromatic amino acids", APPLIED RADIATION AND ISOTOPES, ELSEVIER, OXFORD, GB, Bd. 65, Nr. 11, 17. Oktober 2007 (2007-10-17), Seiten 1227-1231, XP022302102, ISSN: 0969-8043

## Beschreibung

Die Erfindung betrifft ein Verfahren zur Herstellung von L-3,4-Dihydroxy-6-[¹⁸F]fluor-phenylalanin und 2-[¹⁸F]Fluor-L-tyrosin und deren α-methylierten Derivaten aus einem jeweiligen Vorläufer.

Seit zwanzig Jahren ist das PET-Potenzial von aromatischen Aminosäuren, insbesondere von L-3,4-Dihydroxy-6-[¹⁸F]fluor-phenylalanin (=[¹⁸F]FDOPA), bekannt und dieses vor allem für die Untersuchung von Parkinson-Patienten sowie zusätzlich in den letzten Jahren für die Tumordiagnostik (Aminosäuretransport) international auch klinisch etabliert. Insgesamt besteht für neurologische sowie onkologische Anwendungen unverändert ein großes Interesse an einfachen Verfahren zur Synthese von [¹⁸F]FDOPA durch Umsetzung mit dem leicht herstellbaren [¹⁸F]Fluorid.

Aus dem Jahre 2001 ist eine Veröffentlichung von T. Tierling, K. Hamacher und H.H. Coenen mit dem Titel "A new nucleophilic asymetric synthesis of 6-[18F]Fluoro-DOPA" bekannt, die in J. Label. Compds. Radiopharm. 44, Suppl. 1 erschienen ist und in der ein Verfahren zur Herstellung eines [¹⁸F]FDOPA-Vorläufers und dessen Umsetzung zu L-6-[¹⁸F]Fluor-DOPA beschreibt. Nach diesem Verfahren wird ein Vorläufer durch nukleophile Substitution mit einem K¹⁸F Kryptanden zunächst zu Vorläufern und anschließend zu [¹⁸F]FDOPA umgesetzt. Das Produkt wird in einer Enantiomerenreinheit von 85% erhalten.

Gegenwärtige vielstufige nukleophile Synthesewege setzen einen hohen zeitlichen und apparativen Aufwand voraus. Im bisher bekannten direkten nukleophilen Markierungsverfahren konnten lediglich Enantiomerenreinheiten von 85% erhalten werden, wie bei der Synthese von Tierling, Hamacher und Coenen. Elektrophile Reaktionsansätze erlauben nur Radiosynthesen mit ca. zehnfach geringeren Aktivitätsmengen bei relativ hohem Kostenaufwand der Radionuklidherstellung, da die Produktionsraten des für die elektrophile Radiofluorierung notwendigen elementaren [¹⁸F]F₂ bei gleichen Bestrahlungskosten wesentlich geringer sind.

Die Veröffentlichung "Fluorination of aromatic compounds from 1-aryl-3,3-dimethyltriazenes and fluoride anions in acidic medium 2. Synthesis of (S)- [18F]-3-fluoro-α-methyl-phenylalanine" von Thierry Pages et. al. im Journal of Fluorine Chemistry 107 (2001) S. 329-335, offenbart Vorläuferverbindungen und Verfahren zur Herstellung von [¹⁸F] fluormarkierten aromatischen L-Aminosäuren.

Die vielstufigen nucleophilen Verfahren sind schlecht automatisierbar und störanfällig. Die nach dem Stand der Technik bekannten Verfahren zur Herstellung von [¹⁸F]FDOPA- und [¹⁸F]FTyr-Vorläufern führen zu relativ geringen radiochemischen Ausbeuten und sind mit hohem Kostenaufwand verbunden.

Die Veröffentlichung "Synthesis of a Chiral Precursor for No-Carrier-Added (NCA) PET Tracer 6-[18F]Fluoro-L-dopa Based on Regio- and Enantioselective Alkylation of 2,4-Bis (chloromethyl)-5-iodoanisole" von Chiaki Kuroda et al. in der Zeitschrift Bull. Chem. Soc. Jpn. 73, 417-422 aus dem Jahr 2002 offenbart eine Synthese von zyklischen Vorläufern für Radiopharmaka, die einen Jodsubstituenten als Abgangsgruppe haben.

Die Veröffentlichung von Bin Shen et. al "Decarbonylation of multi-substituted [18F]benzaldehydes for modelling syntheses of 18F-labelled aromatic amino acids" aus Applied Radiation and Isotopes, Elsevier, Oxford, GB, Bd. 65 Nr. 11, 17 Oktober 2007 (2007-10-17 / S. 1227-1231 zeigt die Verwendung eines Phasentransferkatalysators bei der ¹⁸F-Fluoridierung.

Es ist daher die Aufgabe der Erfindung, ein Verfahren zur Herstellung von [¹⁸F]FDOPA, [¹⁸F]FTyr und deren α-methylierten Derivate sowie deren Vorläufer und ein Verfahren zur Herstellung deren Vorläufer zur Verfügung zu stellen, welches zu größeren radiochemischen Ausbeuten und zu einer größeren Enantiomerenreinheit führt. Außerdem soll das Verfahren für einen automatisierten Synthesebetrieb geeignet sein.

Die Aufgabe wird erfindungsgemäß gelöst mit den Merkmalen des Anspruchs 1.

Mit den erfindungsgemäßen Verfahren und den Vorläufern ist es nunmehr möglich, [¹⁸F]FDOPA, [¹⁸F]FTyr und deren α-methylierte Derivate in nur drei radioaktiven Schritten enantiomerenrein herzustellen. Die Synthese kann automatisiert durchgeführt werden und führt zu Enantiomerenreiheiten von ≥ 98 %.

Die Figuren zeigen Reaktionschemata für die Herstellung der erfindungsgemäßen Vorläufer sowie für die Herstellung der Zielverbindungen [¹⁸F]FDOPA und [¹⁸F]FTyr und deren α-methylierten Derivate.

Es zeigt:
- Fig.1:: Ein allgemeines Reaktionsschema für die Herstellung des Vorläufers.

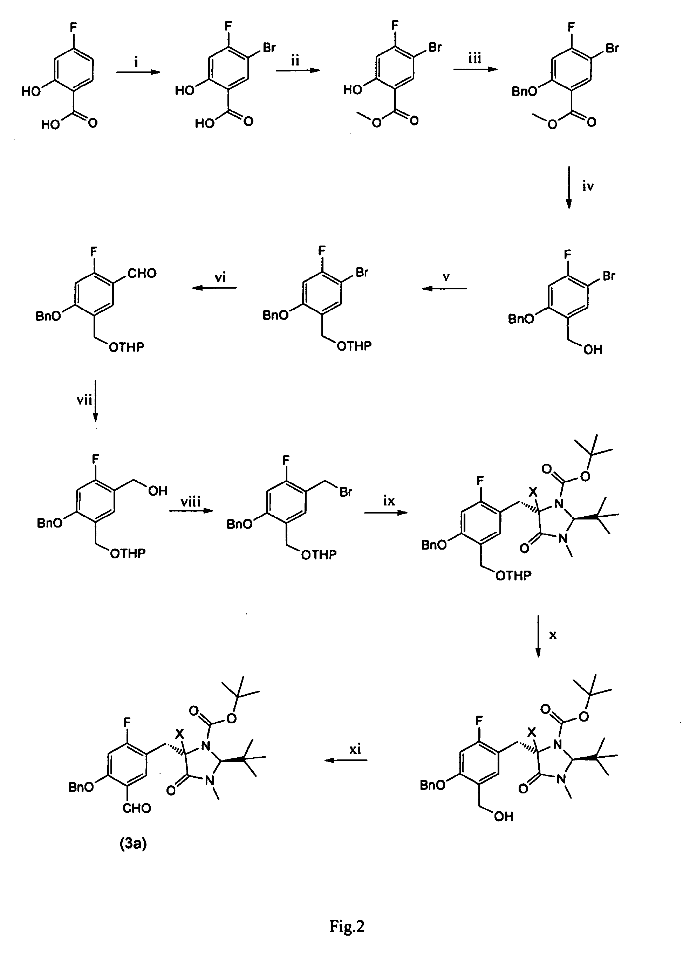

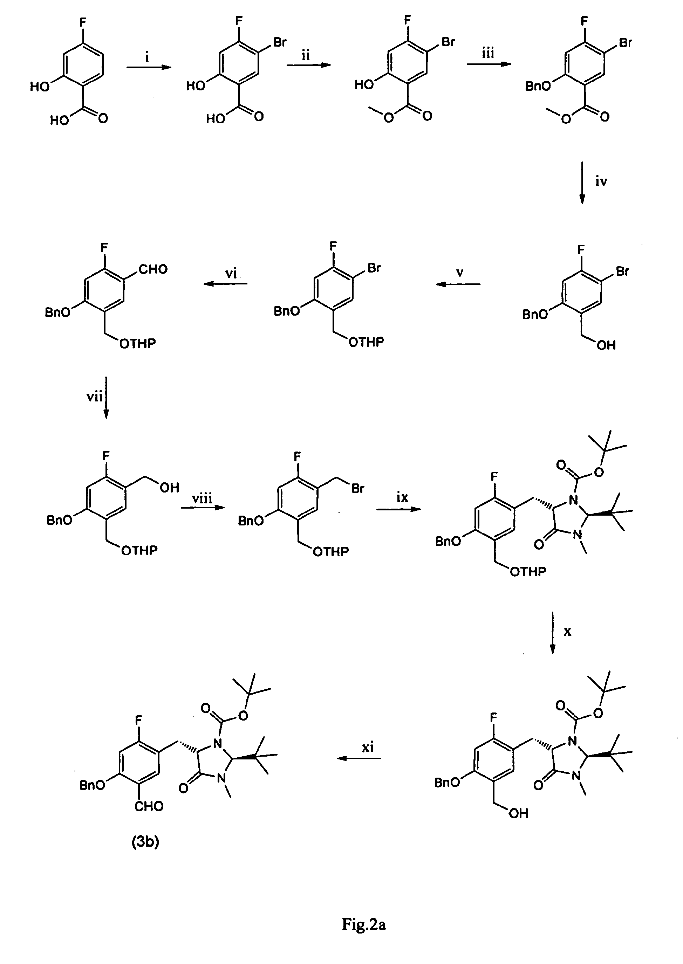

- Fig. 2:: Ein Reaktionsschema für die Synthese des Vorläufers, mit einzelnen Reaktionsschritten.
- Fig.2a:: Ein spezielles Ausführungsbeispiel für die Synthese des Vorläufers.

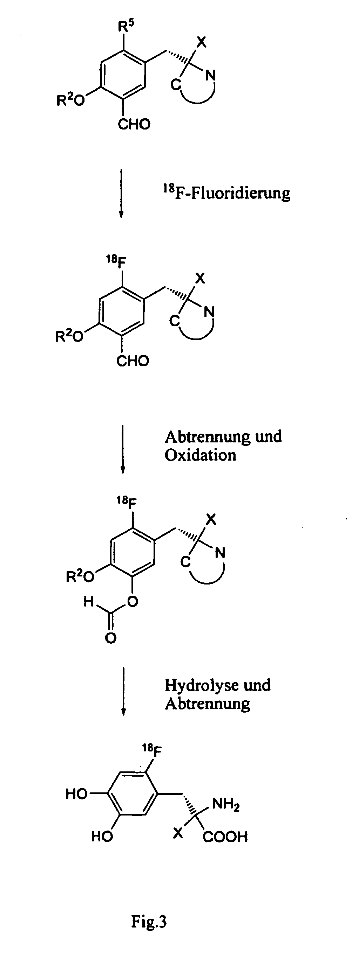

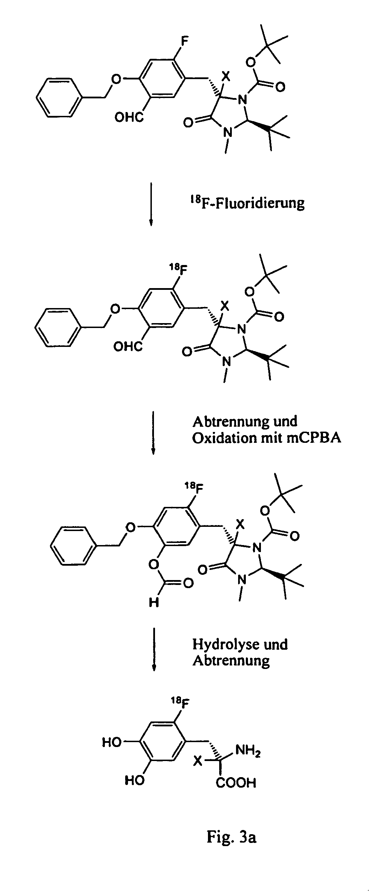

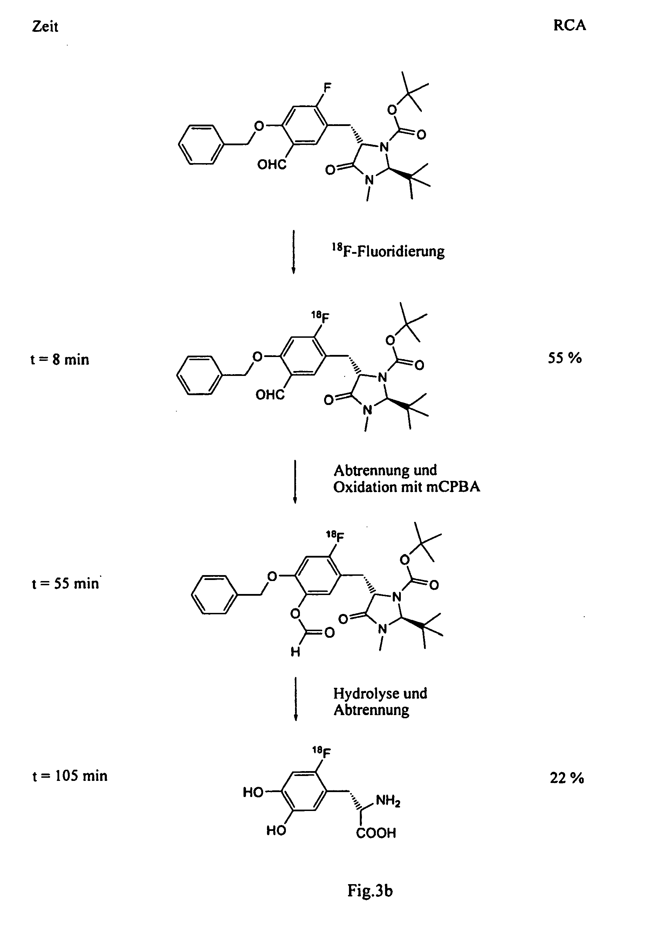

- Fig.3:: Allgemeine Schritte zur Herstellung von [¹⁸F]FDOPA und des
α -methylierten Derivates.
- Fig.3a:: Beispielhafte Herstellung von [¹⁸F]FDOPA und des α-methylierten Derivates.
- Fig.3b:: Schritte zur Herstellung von [¹⁸F]FDOPA (Beispiel)

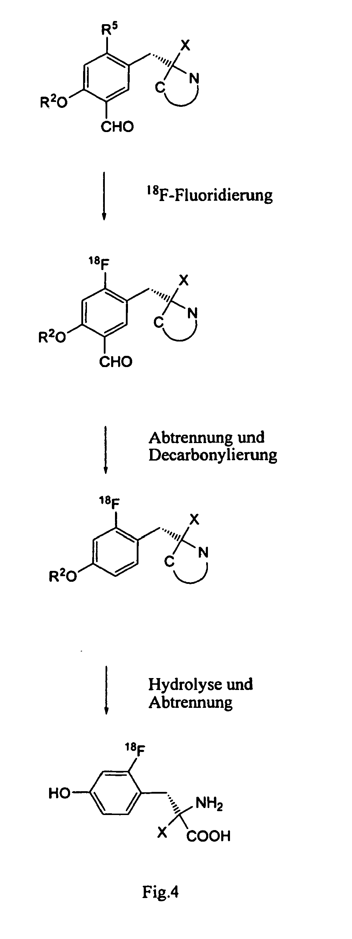

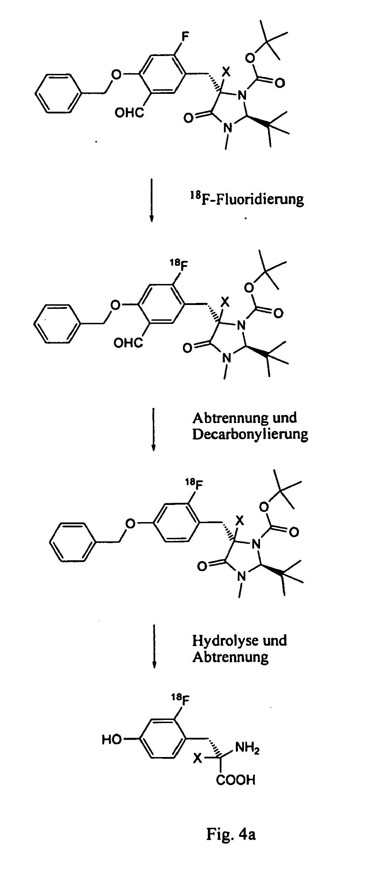

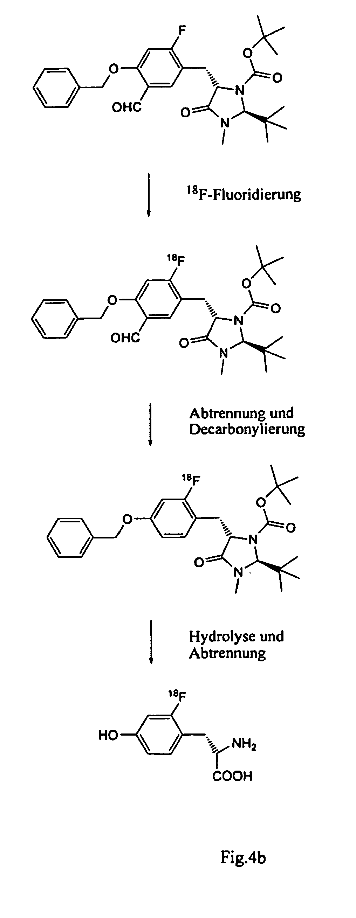

- Fig.4:: Allgemeine Schritte zur Herstellung von [¹⁸F]FTyr und des α-methylierten Derivates.
- Fig.4a:: Beispielhafte Herstellung von [¹⁸F]FTyr und des α-methylierten Derivates.
- Fig.4b:: Schritte zur Herstellung von L-[¹⁸F]FTyr (Beispiel)

Formel 1 zeigt die Struktur von [¹⁸F]FDOPA und des α-methylierten Derivates. In ihr ist X = H oder CH₃.

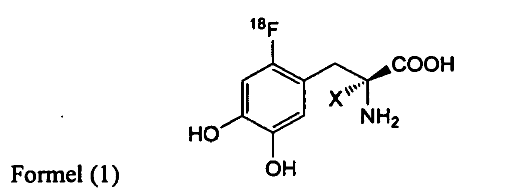

Formel 2 zeigt die Struktur von [¹⁸F]FTyr und des α-methylierten Derivates. In ihr ist X = H oder CH₃. 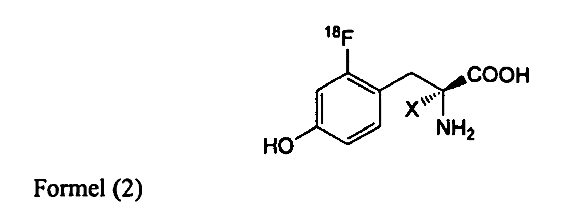 Formel (3) zeigt die Struktur des Vorläufers. In ihr ist X = H oder CH₃.

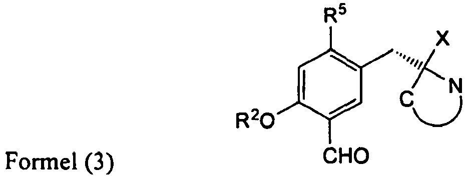

Im Folgenden soll die Erfindung in ihrer allgemeinen Form beschrieben werden:

In den Formeln sowie in den Figuren können als Substituenten Rⁿ, X, folgende Gruppen angegeben werden:
- R¹: = Br, I
- R²: = Benzyl (Bn),
- R³: = Tetrahydropyranyl (THP), Methylthiomethyl (MTM), Methoxymethyl (MOM), TBDMS, TBDPS, allgemein Silylschutzgruppen
- R⁴: = (S)-BOC-BMI: (S)-1-(tert.-Butoxycarbonyl)-2-tert.-butyl-3-methyl-4-imidazolidinon,
Formelschreibweise:
R⁴= 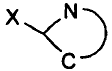
- R⁵: = nukleophile Abgangsgruppe, z. B. F, mit R = Alkyl, z. B. CH₃, C₂H₅
- X: = H oder CH₃

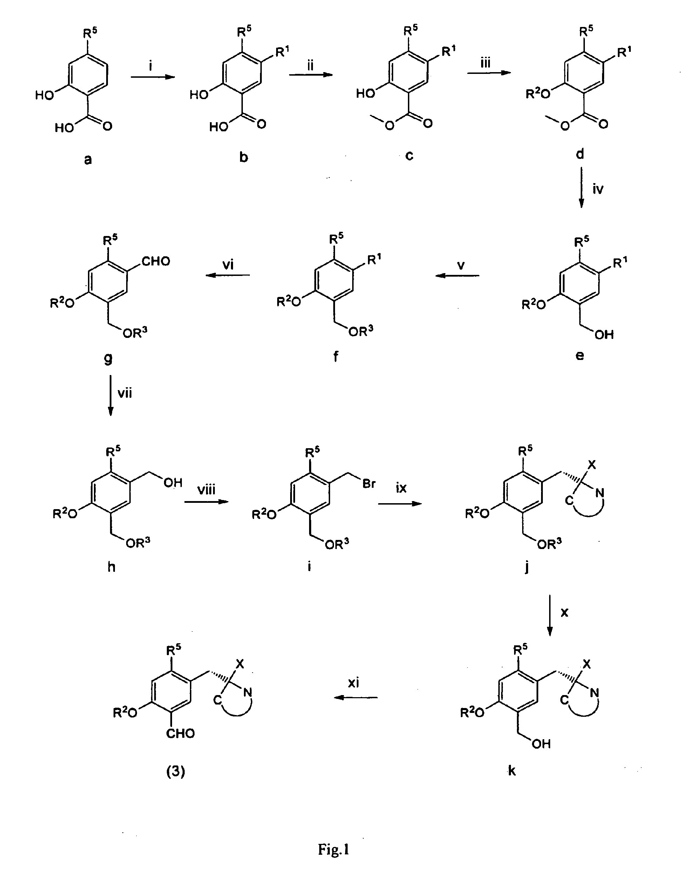

In Figur 1 ist ein allgemeines Reaktionsschema angegeben, nach dem der Vorläufer für [¹⁸F]FDOPA und [¹⁸F]FTyr gemäß Formel 3 hergestellt werden kann.

In Vorbereitung der Synthese des erfindungsgemäßen Vorläufers wird eine Verbindung gemäß Formel a gemäß Reaktionsschritt i zu dem Produkt b halogeniert.

Als Halogene können insbesondere Brom oder Jod eingesetzt werden.

Die Halogenierung kann in einem Temperaturbereich zwischen -20°C und -80°C, vorzugsweise zwischen -50°C und -80°C, besonders bevorzugt bei -78°C, also Trockeneistemperatur durchgeführt werden.

Vorzugsweise erfolgt die Halogenierung unter Zusatz eines Puffers.

Als Puffer kommt beispielsweise Natriumacetat in Betracht.

Als Lösungsmittel kann Methanol, Ethanol oder Essigsäure ohne Puffer eingesetzt werden.

In einem weiteren Schritt ii wird die Verbindung der Formel b an der Carbonsäuregruppe geschützt, vorzugsweise verestert. Vorzugsweise kann eine Veresterung mit einer Methylgruppe erfolgen. Hierzu kann beispielsweise Trimethylsilyldiazomethan als Methylierungsreagenz eingesetzt werden.

Als Lösungsmittel kann dabei mindestens eine Komponente aus der Gruppe bestehend aus Methanol oder Chloroform eingesetzt werden.

Bevorzugt wird ein Gemisch aus Methanol und Chloroform eingesetzt. Dabei ist ein Mischungsverhältnis von 1/5 besonders bevorzugt.

Die Reaktion kann bei Raumtemperatur durchgeführt werden.

Alternativ kann auch eine Veresterung zu einem Ethylester erfolgen.

Die OH-Gruppe des Esters der Verbindung nach Formel c wird in Schritt iii mit einer Schutzgruppe versehen.

Als Schutzgruppen können Benzylgruppen oder Methylgruppen eingesetzt werden.

Die Einführung einer Methylgruppe als Schutzgruppe kann durch eine Umsetzung mit Methyliodid oder Methylbromid erfolgen.

Die Einführung einer Benzylgruppe kann mittels Benzylbromid oder Benzyliodid erfolgen.

Die Wahl des Lösungsmittels ist grundsätzlich frei. Jedoch kann z. B. Aceton oder ein halogenierter Kohlenwasserstoff, wie Chlormethan, Methylenchlorid oder Chloroform eingesetzt werden.

Die Reaktion erfolgt vorzugsweise unter Rückfluss.

Vorzugsweise erfolgt die Umsetzung in Anwesenheit einer Base, wie beispielsweise Kaliumcarbonat oder Aminen, wie primäre, sekundäre oder tertiäre Amine oder NaH.

Die Wahl des Lösungsmittels ist frei. Beispielsweise kann Aceton oder ein halogenierter Kohlenwasserstoff, wie Chlormethan, Methylenchlorid oder Chloroform eingesetzt werden.

Vorzugsweise erfolgt die Umsetzung in Anwesenheit einer Base, wie beispielsweise Kaliumcarbonat.

Mit diesem Herstellungsverfahren kann die Verbindung d als Edukt für das erfindungsgemäße Herstellungsverfahren des Vorläufers beispielhaft hergestellt werden. Jedoch können für d auch andere Synthesewege eingeschlagen werden.

Die Estergruppe von Verbindung d wird nun erfindungsgemäß reduziert.

Die Reduktion kann beispielsweise mit einem Hydrid, besonders bevorzugt mit Lithiumaluminiumhydrid durchgeführt werden.

Vorzugsweise erfolgt die Hydrierung bei Raumtemperatur.

Als Lösungsmittel kann ein Ether, beispielsweise Diethylether oder THF eingesetzt werden. Vorzugsweise wird Diethylether als Lösungsmittel verwendet.

Der resultierende Alkohol e wird in Schritt v mit einer Schutzgruppe geschützt.

Als Schutzgruppen kann THP, MTM oder MOM eingesetzt werden.

Hierzu kann p-Toluolsulfonsäure als Katalysator hinzu gegeben werden.

Als Lösungsmittel kann Dichlormethan oder Tetrahydrofuran eingesetzt werden.

Die Reaktionstemperatur liegt vorzugsweise zwischen 0°C und Raumtemperatur, beispielsweise 17°C bis 25°C.

In einem folgenden Schritt vi wird bei Verbindung f der Substituent R¹ durch eine Formylgruppe ersetzt.

Die Formylierung kann dabei beispielsweise mit Anilid, Formylpiperidin oder Dimethylformamid in Anwesenheit von Metallierungsreagentien, wie sec.-Butyllithium, n-Buyllithium, tert.-Butyllitium, Lithium oder Magnesium erfolgen.

Als Lösungsmittel kann Tetrahydrofuran oder beispielsweise ein anderer Ether eingesetzt werden.

Die Reaktion kann in einem Temperaturbereich zwischen -20°C und -80°C, vorzugsweise zwischen -50°C und -80°C besonders bevorzugt bei -78°C, also Trockeneistemperatur durchgeführt werden.

Die resultierende Verbindung g wird in einem folgenden Schritt vii zu einem Alkohol h reduziert.

Als Reduktionsmittel kann beispielsweise ein Metallhydrid, wie Natriumborhydrid, Lithiumaluminiumhydrid eingesetzt werden.

Geeignete Lösungsmittel sind insbesondere bei der Verwendung von Natriumborhydrid Methanol oder auch andere Alkohole.

Vorzugsweise wird die Reaktion bei Raumtemperatur durchgeführt.

Der Alkohol h wird in der Folgereaktion viii zu Verbindung i halogeniert oder tosyliert.

Hierzu können vorzugsweise Tetrabrommethan in Gegenwart von Triphenylphosphin als Sauerstofffänger eingesetzt werden.

Als Lösungsmittel können Dichlormethan oder allgemein halogenierte Kohlenwasserstoffe eingesetzt werden.

Die bevorzugte Temperatur liegt bei 0°C bis ca. 4°C.

Verbindung i wird in Reaktionsschritt ix mit einem chiralen Aminosäurereagenz umgesetzt. Hierzu wird Verbindung i mit (S)-BOC-BMI: (S)-1-(tert.-Butoxycarbonyl)-2-tert.-butyl-3-methyl-4-imidazolidinon, umgesetzt. Die Umsetzung kann in Anwesenheit von Lithiumdiisopropylamin erfolgen.

Als Lösungsmittel kann Tetrahydrofuran oder ein Ether, vorzugsweise Diethylether, mindestens eine Komponente davon eingesetzt werden.

Die resultierende Verbindung j wird in Schritt x an der Funktion OR³ entschützt.

Hierzu kann beispielsweise Pyridinium-p-toluolsulfonsäure verwendet werden. Jedoch kann jedes bekannte Verfahren zur Entfernung der Schutzgruppe eingesetzt werden, wie beispielsweise der Einsatz von Säuren oder MgBr₂.

Als Lösungsmittel kommen Alkohole, wie Ethanol in Betracht.

Das Reaktionsprodukt k wird zu einem Aldehyd oxidiert.

Hierzu können bekannte, milde Oxidationsverfahren eingesetzt werden.

Die Reaktion findet in einem Bereich von -20°C bis -80°C, -30°C -bis -80°C oder bevorzugt, zwischen -50°C bis -80°C statt. Typischerweise bei Trockeneistemperatur von ca. -78°C.

Hierzu kann beispielhaft eine Oxidation nach Swern durchgeführt werden. Die Umsetzung erfolgt mit Oxalylchlorid, Dimethylsulfoxid in Anwesenheit von Triethylamin.

Als Lösungsmittel kann ein halogenierter Kohlenwasserstoff, wie Dichlormethan verwendet werden.

Reaktionsprodukt ist der erfindungsgemäße Vorläufer nach Formel 3.

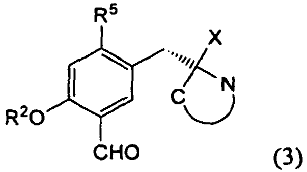

In einer weiteren Umsetzung kann der Vorläufer nach Formel 3 zu [¹⁸F]FDOPA oder [¹⁸F]FTyr umgesetzt werden.

Hierzu wird das Fluoratom des Vorläufers gemäß Formel 3 durch ¹⁸F-substituiert.

Diese Fluoridierung kann durch Standardverfahren erfolgen. Dabei werden die Phasentransferkatalysatoren Kryptofix-Kaliumoxalat oder Tetrabutylammoniumhydrogencarbonat erfindungsgemäß als Anionenaktivator für [¹⁸F⁻]Fluorid eingesetzt.

Das ¹⁸F-fluoridierte Zwischenprodukt wird in einem weiteren Schritt abgetrennt und im Fall des [¹⁸F]FDOPA und des α-methylierten Derivates zu einem Ester oxidiert.

Die Abtrennung kann durch Festphasenextraktion erfolgen. Hierzu wird das Reaktionsgemisch über eine Reverse-phase-Kartusche gereinigt.

Die Oxidation der Aldehydgruppe kann mit mCPBA, oder Peressigsäure oder Perborat durchgeführt werden. Als Lösungsmittel können halogenierte Kohlenwasserstoffe, wie Chloroform oder Methylenchlorid eingesetzt werden.

Im Falle des [¹⁸F]FTyr oder dessen α-methylierten Derivates wird an Stelle der Oxidation eine Decarbonylierung durchgeführt.

Geeignete Katalysatoren für die Decarbonylierung enthalten zweckmäßig vorzugsweise ein oder mehrere Übergangsmetalle der I., II., VI., VII. und VIII. Nebengruppe, wie Chrom, Mangan, Nickel, Kupfer oder Zink, bevorzugt jedoch ein oder mehrere Metalle aus der Gruppe der Platin-Metalle, insbesondere Rhodium. Dabei kann im heterogenen System mit festen, auf Trägern befindlichen Katalysatoren oder im homogenen System in flüssiger Phase gearbeitet werden.

Lösliche Rhodium-Komplexe, mit denen in einem homogenen flüssigen System gearbeitet werden kann oder mit denen Träger imprägniert werden können sind beispielsweise Rhodium(I)-Komplexe, wie ClRh(PPh₃)₃ ("Wilkinson- Katalysator" ) , ClRh(CO) (PPh₃)₂, [ClRh(CO)₂]₂, acacRh(CO) (PPh₃) , acacRh(CO)₂, (C₅H₅)Rh(C₈H₁₄) und (C₃H₅)Rh(PPh₃), wobei Ph für Phenyl, acac für Acetylacetonat, C₈H₁₄ für Cycloocten, C₅H₅ für Cyclopentadienyl und C₃H₅ für Allyl stehen. Geeignet sind auch Rhodium(II)- und Rhodium(III)-Komplexe, wie Rhodium(II)-acetat, Rhodium(II)-2,4-difluor-benzoat, Rh(acac)₃, RhCl₃'xH₂0, Rh(NO₃)₃ und (C₃H₅)RhCl₂(PPh₃)₂. Zu diesen Rhodium-Komplexen können vorteilhaft noch Verbindungen, die als Liganden wirken können, wie Phosphane, Phosphite oder Amine, zugesetzt werden.

In einem weiteren Schritt wird der erhaltene Ester einer Hydrolyse unterzogen, wobei [¹⁸F]FDOPA oder [¹⁸F]FTyr oder deren α-methyliertes Derivat erhalten wird.

Die Hydrolyse kann in wässriger, vorzugsweise konzentrierter HI oder HBr oder in einer Lösung mit KI und HBr durchgeführt werden.

Eine Abtrennung des Produktes ist durch HPLC möglich.

Durch Einsatz des Markierungsvorläufers nach Formel (3) gelingt die Darstellung eines enantiomerenreinen Produktes nach Formel 3 (ee ≥ 98%) über eine nukleophile Synthese mit nur drei radioaktiven Schritten. Damit wird eine automatisierte Routinesynthese praktikabel. Die radiochemischen Ausbeuten liegen bei 22%. Auch die Endprodukte [¹⁸F]FDOPA und [¹⁸F]FTyr können in einer Enantiomerenreinheit von ≥ 98% erhalten werden.

Das erfindungsgemäße Verfahren mit dem Vorläufer nach Formel 3 ermöglicht eine Synthese, die automatisiert zu betreiben ist.

Apparaturen für den automatisierten Synthesebetrieb umfassen in der Regel ein Vorlagegefäß, in welches aus Vorratsgefäßen, welche mit dem Vorlagegefäß über Zuleitungen in Verbindung stehen über eine Steuerung mit Reagentien beschickt wird. Die Befüllung und Entleerung des Vorlagegefäßes erfolgt in der Regel durch Erzeugung eines Überdrucks oder eines Unterdrucks. Beispielhaft kann die kommerziell verfügbare Vorrichtung TRACERlab FX F-N genannt werden, welche neben Vorratsgefäßen für Reagentien, einem Vorlagegefäß aus Glaskohlenstoff und Magnetrührer sowie herausziehbarer Nadel, mit einem Aktivitätsdetektor sowie einem Vakuumsystem mit Kühlfalle ausgestattet ist. Die Vorrichtung besitzt eine [¹⁸O]-Wasser-Aufarbeitung sowie eine Festphasenextraktionseinheit mit präparativer HPLC, zwei HPLC-Eluenten und HPLC Flusskontrolle, UV und Radioaktivitätsdetektoren für die HPLC, Auffangbehälter für Fraktionen, Festphasenextraktion sowie eine HPLC-Lösungsmittelrückführung. Ähnliche Vorrichtungen sind aus "One-step high-radiochemical-yield synthesis of [18F]FP-CIT using a protic solvent system" in Nuc. Med. Biol., 2007; 34: 345-351 von S. Lee, S Oh, D.Chi, S. Kang, H. Kil, J Kim, D. Moon und weiterhin von Chen X, Park r, Shahinian AH, et al. 18F-labeled RGD peptide: initial evaluation for imaging brain tumor angiogenesis /Nuc. Med. Biol. 2004; 31: 179-189 bekannt.

Der Vorläufer ermöglicht eine vollautomatisierte Umsetzung einer solchen Anlage, in dem eine ¹⁸F-Fluoridierung des Vorläufers nach Formel 3 durchgeführt wird und danach die Aufarbeitung zum Endprodukt, nämlich zu [¹⁸F]FDOPA oder [¹⁸F]FTyr erfolgt. [¹⁸F]FDOPA und [¹⁸F]FTyr werden in einer Enantiomerenreinheit von ≥ 98% erhalten.

### Beispiel I:

Die Synthese gemäß der Figur 1 kann mit folgenden Reagentien durchgeführt werden:
i) Methanol, Natriumacetat, Brom
ii) Methanol, Chloroform, Trimethylsilyldiazomethan
iii) Aceton, Kaliumcarbonat, Methyljodid
iv) Diethylether, Lithiumaluminiumhydrid
v) Dichlormethan, Dihydropyan, p-Toluolsulfonsäure
vi) Tetrahydropyran, sec.-Buthyllithium, Dimethylformamid
vii) Methanol, Natriumborhydrid
viii) Dichlormethan, Tetrabrommethan, Triphenylphosphin
ix) Tetrahydropyran, Lithiumdiisopropylamin, (S)-Boc-BMI
x) Ethanol, Pyridiunium-p-Toluolsulfonsäure
xi) Dichlormethan, Oxalylchlorid, Dimethylsulfoxid, Triethylamin

### Spezielles Ausführungsbeispiel:

5-Brom-4-fluor-2-hydroxybenzoesäure
1 g (6,4 mmol) 4-Fluor-2-hydroxybenzoesäure und 2,2 g (26,88 mmol) Natriumacetat werden in 10 ml absolutem Methanol gelöst und auf -70°C gekühlt. Nach Zugabe von 0,33 ml (6,4 mmol) Brom in 10 ml Methanol in 30 min, wird die Reaktionslösung auf RT erwärmt, das Lösungsmittel im Vakuum entfernt und der Rückstand in verdünnter Salzsäure aufgenommen. Der entstandene Niederschlag wird abgesaugt, mit Wasser gewaschen und in Essigsäureethylester aufgenommen. Nach Trocknung mit Natriumsulfat wird das Lösungsmittel im Vakuum entfernt und das reine Produkt erhalten.

| | |
|---|---|
| Form: | farbloser Feststoff |
| Ausbeute: | 1,23 g (5,2 mmol; 82 %) |
| R_{f}: | 0,50 (Chloroform/Methanol = 2:1 + 0,1 % TEA) |

Methyl-5-brom-4-fluor-2-hydroxybenzoat

In 10 ml wasserfreiem Dichlormethan und 2 ml absolutem Methanol werden 1,23 g (5,2 mmol) 5-Brom-4-fluor-2-hydroxybenzoesäure gelöst, mit 3,4 ml (6,76 mmol) Trimethylsilyldiazomethan versetzt und 30 min bei RT gerührt. Die flüchtigen Bestandteile werden im Vakuum entfernt und das Produkt in reiner Form erhalten.

| | |
|---|---|
| Form: | farbloser Feststoff |
| Ausbeute: | 1,27 g (5,1 mmol; 99 %) |
| R_{f}: | 0,85 (Diethylether/Petrolether= 1:2) |

Methyl-2-(benzyloxy)-5-brom-4-fluorbenzoat

Eine Suspension von 12,7 g (51 mmol) Methyl-5-brom-4-fluor-2-hydroxybenzoat, 6,7 ml (56,1 mmol) Benzylbromid und 14,01 g (102 mmol) Kaliumcarbonat in 100 ml Aceton wird 12 h unter Rückfluss erhitzt. Nach Abkühlung wird das Kaliumcarbonat filtriert und mit Aceton gewaschen, das Lösungsmittel im Vakuum entfernt und Rohprodukt säulenchromatographisch an Kieselgel unter Verwendung von Diethylether/Petrolether 1:3 gereinigt.

| | |
|---|---|
| Form: | farbloser Feststoff |
| Ausbeute: | 13,14 g (38,76 mmol; 76 %) |
| R_{f}: | 0,58 (Diethylether/Petrolether = 1:3) |

[2-(Benzyloxy)-5-brom-4-fluorphenyl]methanol

Zu einer Lösung von 13,14 g (38,76 mmol) Methyl-2-(benzyloxy)-5-brom-4-fluorbenzoat in 100 ml wasserfreiem Diethylether werden unter Argonatmosphäre 23,25 ml (23,25 mmol) Lithiumaluminiumhydrid-Lösung (IM in Diethylether) langsam zugetropft. Danach erhitzt man 60 min unter Rückfluss, kühlt ab, gießt die Reaktionslösung in Eiswasser und säuert an, bis sich der Niederschlag löst. Das Reaktionsgemisch wird mit Diethylether extrahiert, mit Natriumchlorid gewaschen und über Natriumsulfat getrocknet. Nach Entfernung des Lösungsmittels wird das reine Produkt erhalten.

| | |
|---|---|
| Form: | farbloser Feststoff |
| Ausbeute: | 10,25 g (32,95 mmol; 85%) |
| R_{f}: | 0,58 (Diethylether/Petrolether = 1:3) |

2-[2-(Benzyloxy)5-brom-4-fluorbenzyloxy]tetrahydro-2H-pyran

Eine Lösung von 10,25 g (32,95 mmol) [2-(Benzyloxy)-5-brom-4-fluorphenyl]methanol und 1,9 ml (20,5 mmol) 3,4-Dihydro-2H-pyran in Dichlormethan wird bei 0°C mit einer Spatelspitze Toluolsulfonsäure Monohydrat versetzt und 45 min gerührt. Die Reaktionslösung wird mit Diethylether versetzt, mit Natriumchlorid- und Natriumcarbonat-Lösung sowie Wasser und nochmals mit Natriumchlorid-Lösung gewaschen, über Natriumsulfat getrocknet und das Lösungsmittel im Vakuum entfernt.

| | |
|---|---|
| Form: | farbloses Öl |
| Ausbeute: | 12,76 g (32,29 mmol; 98 %) |
| R_{f}: | 0,75 (Diethylether/Petrolether = 1:2) |

4-(Benzyloxy)-2-fluor-5-[tetrahydro-2H-pyran-2-yloxy]methyl)benzaldehyd

12,76 g (32,29 mmol) 2-[2-(Benzyloxy)5-brom-4-fluorbenzyloxy]tetrahydro-2H-pyran werden in 70 ml absolutem Tetrahydrofuran gelöst, bei -78°C unter Argonatmosphäre langsam mit 24,2 ml (33,9 mmol) sec.-BuLi (1,4 M in Cyclohexan) versetzt und 45 min gerührt. Nach Zugabe von 3,5 ml (45,21 mmol) Dimethylformamid rührt man die Reaktionslösung weitere 60 min bei RT. Nach Zugabe von Wasser wird mit Diethylether extrahiert, die organische Phase über Natriumsulfat getrocknet und das Lösungsmittel im Vakuum entfernt. Das Rohprodukt wird an Kieselgel mit Diethylether/Petrolether 1:3 chromatographiert.

| | |
|---|---|
| Form: | farbloser Feststoff |
| Ausbeute: | 4,33 g (12,59 mmol; 39 %) |
| R_{f}: | 0,53 (Diethylether/Petrolether = 1:3) |

[4-Benzyloxy-2-fluor-5-(tetrahydro-pyran-2-yloxymethyl)-phenyl]methanol

Eine Lösung von 4,33 g (12,59 mmol) 4-(Benzyloxy)-2-fluor-5-[tetrahydro-2H-pyran-2-yloxy]methyl)benzaldehyd in 50 ml wasserfreiem Methanol wird unter Rühren portionsweise mit 0,71 g (18,89 mmol) Natriumborhydrid versetzt und 30 min bei RT gerührt. Nach Zugabe von Wasser wird mit Diethylether extrahiert, die organische Phase über Natriumsulfat getrocknet und das Lösungsmittel im Vakuum entfernt.

| | |
|---|---|
| Form: | farbloses Öl |
| Ausbeute: | 4,27 g (12,34 mmol; 98 %) |
| R_{f}: | 0,43 (Diethylether/Petrolether = 1:1) |

2-(2-Benzyloxy-5-brommethyl-4-fluor-benzyloxy-tetrahydro-pyran

Zu einer eiskalten Lösung von 4,27 g (12,34 mmol) [4-Benzyloxy-2-fluor-5-(tetrahydropyran-2-yloxymethyl)-phenyl]methanol und 5,11 g (15,42 mmol) Tetrabrommethan in 100 ml wasserfreiem Dichlormethan werden langsam 4,53 g (17,27 mmol) Triphenylphosphin über 10 min zugegeben und 45 min gerührt. Die Reaktionslösung wird mit Pentan versetzt, der Niederschlag abgesaugt und mit Dichlormethan gewaschen. Das Filtrat wird mit 5% Natriumhydrogencarbonatlösung, Wasser und Natriumchloridlösung gewaschen, über Magnesiumsulfat getrocknet. Nach der Entfernung des Lösungsmittels wird der Rückstand säulenchromatographisch an Kieselgel mit Diethylether/Petrolether 1:5 gereinigt.

| | |
|---|---|
| Form: | farbloses Öl |
| Ausbeute: | 1,95 g (4,77 mmol; 39 %) |
| R_{f}: | 0,67 (Diethylether/Petrolether = 1:5) |

tert.-Butyl-5{4-(benzyloxy)-2-fluor-5-[(tetrahydro-2H-pyran-2-yloxy)methyl]benzyl-2-tert.-butyl-3-methyl-4-oxoimodazolidin-1-carboxylat

Zu einer Lösung von 1,22 g (4,77 mmol) BOC - BMI in wasserfreiem Diethylether werden bei -78° C unter Argonatmosphäre 3,2 ml (4,77 mmol, 1,5 M in THF) LDA gegeben und 40 min gerührt. Nach Zugabe von 1,95 g (4,77 mmol) 2-(2-Benzyloxy-5-brommethyl-4-fluor-benzyloxy-tetrahydro-pyran wird die Reaktionslösung 18 Stunden bei RT gerührt, mit gesättigter NH₄CL-Lösung versetzt und in Diethylether und Wasser aufgenommen. Die wässrige Phase wird 2 mal mit Diethylether extrahiert; die vereinigten organischen Extrakte über Na-SO₄ getrocknet und das Lösungsmittel im Vakuum reduziert. Der Rückstand wird anschließend an Kieselgel mit Diethylether/Petrolether 2:1 chromatographiert.

| | |
|---|---|
| Form: | farbloser Schaum |
| Ausbeute: | 0,75 g (1,29 mmol; 27 %) |
| R_{f}: | 0,69 (Diethylether/Petrolether = 2:1) |

tert.-Butyl-5-[4-benzyloxy-2-fluor-5-(hydroxymethyl)benzyl]2-tert.-butyl-3-methyl-4-oxoimidazolidin-1-carboxylat
0,75 g (1,29 mmol) tert.-Butyl-5{4-(benzyloxy)-2-fluor-5-[(tetrahydro-2H-pyran-2-yloxy)methyl]benzyl-2-tert.-butyl-3-methyl-4-oxoimodazolidin-1-carboxylat werden in 30 ml Ethanol gelöst, mit 28 mg (0,13 mmol) PPTS versetzt und 18 h bei 55 °C gerührt. Nach Abkühlung wird das Lösungsmittel entfernt, der Rückstand in Diethylether aufgenommen, die organische Phase mit Natriumchloridlösung gewaschen und über Magnesiumsulfat getrocknet. Das Lösungsmittel wird im Vakuum entfernt und der Rückstand säulenchromatographisch an Kieselgel unter Verwendung von Diethylether/Petrolether 5:1 gereinigt.

| | |
|---|---|
| Form: | farbloser Schaum |
| Ausbeute: | 0,60 g (1,20 mmol; 93 %) |
| R_{f}: | 0,78 (Diethylether/Petrolether = 2: 1) |

tert.-Butyl-5-(4-benzyloxy-2-fluor-formylbenzyl)-2-tert.-butyl-3-methyl-4-oxoimidazolidin-1-carboxylat

Unter Argonatmosphäre werden 0,11 ml (1,32 mmol) Oxalylchlorid in 1 ml Dichlormethan bei -60°C langsam mit 0,2 ml (2,88 mmol) Dimethylsulfoxid versetzt und 10 min gerührt. Nach Zugabe von 0,60 g (1,20 mmol) tert.-Butyl-5-[4-benzyloxy-2-fluor-5-(hydroxymethyl)benzyl]2-tert.-butyl-3-methyl-4-oxoimidazolidin-1-carboxylat in 5 ml Dichlormethan wird weitere 15 min gerührt, die Reaktionslösung mit 0,83 ml (6 mmol) Triethylamin versetzt, langsam auf RT erwärmt und nach Zugabe von 5 ml Wasser für weitere 10 min gerührt. Die wässrige Phase wird abgetrennt und mit Dichlormethan extrahiert. Die organische Phase wird über Natriumsulfat getrocknet, das Lösungsmittel im Vakuum entfernt und der Rückstand an Kiesegel mit Diethylether/Petrolether 2:1 chromatographiert.

| | |
|---|---|
| Form: | farbloser Schaum |
| Ausbeute: | 0,42 g (0,85 mmol; 71 %) |
| R_{f}: | 0,78 (Diethylether/Petrolether = 2: 1) |

### Aktivsynthese

In ein konisch zulaufendes Reaktionsgefäß (Rectivial, 5 ml) mit Magnetrührstäbchen werden zur Trocknung der wässrigen [¹⁸F]Fluoridlösung 130 µl Tetrabutylammoniumhydrogencarbonat in 0,9 ml abs. Acetonitril (für DNA-Synthese, Merck) gegeben. Der Reaktor wird über Schraubkappe mit Silikonseptum verschlossen, durch das zwei Einmalkanülen für den Vakuum- und Argonanschluß gestochen werden. Die Lösung wird anschließend unter vermindertem Druck bei einer Temperatur von 80°C zur Trocknung eingedampft. Diese azeotrope Trocknung wird zweimal mit je 0,8 ml Acetonitril wiederholt und abschließend die Apparatur 5 min evakuiert. Nach Begasung des Reaktors mit Argon wird eine Temperatur von 120°C eingestellt, 23µmol tert.-Butyl-5-(4-benzyloxy-2-fluor-formylbenzyl)-2-tert.-butyl-3-methyl-4-oxoimidazolidin-1-carboxylat in 0,8 ml N,N-Dimethylformamid aufgenommen und mittels einer Tuberkulinspritze in das Reaktionsgefäß überführt. Das Reaktionsgemisch der ¹⁸F-Markierung wird nach 8 min Reaktionszeit zur Abtrennung des Lösungsmittels und des Phasentransferkatalysators in 9 ml Wasser aufgenommen und über eine LiChrolut® RP-18e-Kartusche (500mg) geleitet. Nach dem Spülen der Kartusche mit 5 ml Wasser wird diese mit einem kräftigen Argonstrom 2 min getrocknet. Die fixierten organischen Bestandteile werden mit 1,5 ml Acetonitril in einen Reaktor eluiert und azeotrop getrocknet. Nach Zugabe von 22 mg (92µmol, 77%ig) mCPBA in 1 ml Chloroform wird 20 min bei 60°C gerührt und das Lösungsmittel anschließend entfernt. Der Rückstand wird mit 1 ml 48% HBr versetzt und 30 min auf 150°C erhitzt. Nach Abkühlung wird die HBr-Phase in 1 ml Wasser aufgenommen und das Produkt mittels semipräparativer HPLC gereinigt.

## Patentansprüche

1. Verfahren zur Herstellung einer Verbindung nach Formel (1) und (2) 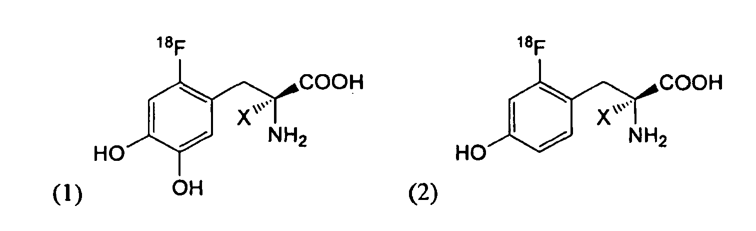 mit X = H oder CH₃
**dadurch gekennzeichnet,**
**dass** die Verbindung nach Formel (3) i 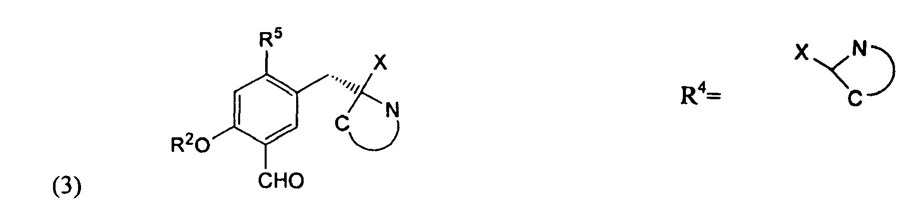 mit X = H oder CH₃ und R⁵= nukleophile Abgangsgruppe, = F; R² = Benzyl (Bn) und R⁴ = (S)-BOC-BMI: (S)-1-(tert.-Butoxycarbonyl)-2-tert.-butyl-3-methyl-4-imidazolidinon ist,
unter Verwendung von Tetrabutylammonium-hydrogencarbonat oder Kryptofix-kaliumoxalat als Phasentransferkatalysator ¹⁸F-fluoridiert wird,
in einem weiteren Schritt abgetrennt und im Fall der Herstellung der Verbindung nach Formel (1) oxidiert wird und im Fall der Herstellung der Verbindung nach Formel (2) decabonyliert wird, das resultierende Produkt hydrolysiert und abgetrennt wird.

2. Verfahren nach Anspruch 1,
**dadurch gekennzeichnet**,
die Abtrennung des ¹⁸F-Fluorierungsproduktes durch Festphasenextraktion erfolgt.

3. Verfahren nach einem der Ansprüche 1 oder 2,
**dadurch gekennzeichnet,**
**dass** die Formylgruppe in Fall der Herstellung der Verbindung nach Formel (1) zu einem Ester oxidiert wird,

4. Verfahren nach Anspruch 3,
**dadurch gekennzeichnet,**
**dass** die Oxidation mit MCPBA, oder Peressigsäure oder Perborat durchgeführt wird.

5. Verfahren nach einem der Ansprüche 1 oder 2,
**dadurch gekennzeichnet,**
**dass** der ¹⁸F-fluoridierte Voräufer für die Herstellung der Verbindung nach Formel (2) mittels eines Katalysators decabonyliert wird.

6. Verfahren nach einem der Ansprüche 1 bis 5,
**dadurch gekennzeichnet,**
**dass** der Ester oder das Decarbonylierungsprodukt hydrolysiert wird.

7. Verfahren nach einem der Ansprüche 1 bis 5,
**dadurch gekennzeichnet,**
**dass** das Produkt nach Formel (1) oder (2) durch HPLC abgetrennt wird.

## Claims

1. Method for producing a compound according to formula (1) and (2) 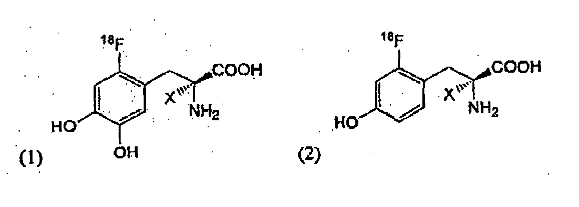 where X = H or CH₃
**characterised in that** the compound according to formula (3) 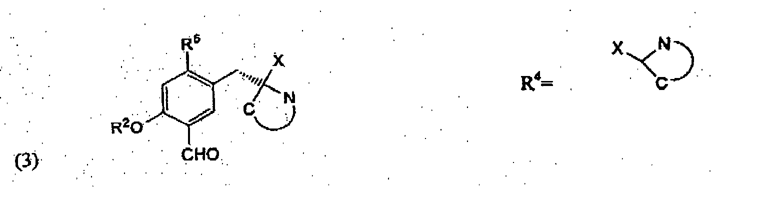 where X = H or CH₃ and R⁵ = nucleophilic leaving group = F; R² = benzyl (Bn) and R⁴ = (S)-BOC-BMI : (S)-1-(tert-butoxycarbonyl)-2-tert-butyl-3-methyl-4-imidazolidinone,
making use of tetrabutylammonium-hydrogen carbonate or cryptofix-potassium oxalate as phase transfer catalyst, is ¹⁸F-fluoridated, in a further step, is separated and, in the case of production of the compound according to formula (1), is oxidised and, in the case of production of the compound according to formula (2), is decarbonylated, the resulting product is hydrolysed and separated.

2. Method according to claim 1, **characterised in that** the separation of the ¹⁸F-fluoridation product is carried out by means of solid-phase extraction.

3. Method according to one of the claims 1 or 2, **characterised in that**, in the case of production of the compound according to formula (1), the formyl group is oxidised to an ester.

4. Method according to claim 3, **characterised in that** the oxidation is carried out with MCPBA or peracetic acid or perborate.

5. Method according to one of the claims 1 or 2, **characterised in that** the ¹⁸F-fluoridated precursor for the production of the compound according to formula (2) is decarbonylated by means of a catalyst.

6. Method according to one of the claims 1 to 5, **characterised in that** the ester or the decarbonylation product is hydrolysed.

7. Method according to one of the claims 1 to 5, **characterised in that** the product according to formula (1) or (2) is separated by means of HPLC.

## Revendications

1. Procédé de production d'un composé de formules (1) et (2) 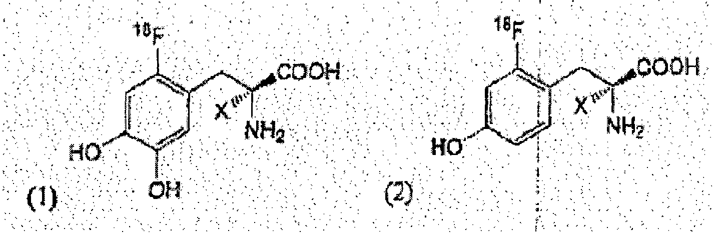 où X = H ou CH₃,
**caractérisé en ce que**
le composé de formule (3) 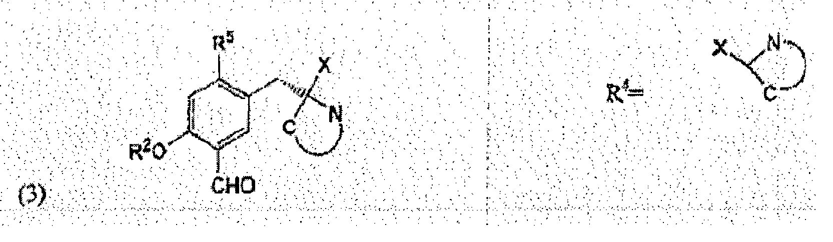 où X = H ou CH₃ et R⁵ = un groupe partant nucléophile, - F ; R² = un groupe benzyle (Bn) et R⁴ = (S)-BOC-BMI : (S)-1-(tert-butoxycarbonyl)-2-tert-butyl-3-méthyl-4-imidazolidinone,
est fluoré au ¹⁸F en utilisant de l'hydrogénocarbonate de tétrabutylammonium ou de l'oxalate de potassium Cryptofix comme catalyseur de transfert de phase,
est séparé dans une autre étape, et dans le cas de la production du composé de formule (1) est oxydé et dans le cas de la production du composé de formule (2) est décarbonylé, le produit obtenu étant hydrolysé et séparé.

2. Procédé selon la revendication 1,
**caractérisé en ce que**
la séparation du produit fluoré au ¹⁸F s'effectue par extraction en phase solide.

3. Procédé selon l'une des revendications 1 ou 2,
**caractérisé en ce que**
le groupe formyle est oxydé en un ester dans la cas de la production du composé de formule (1).

4. Procédé selon la revendication 3,
**caractérisé en ce que**
l'oxydation est réalisée avec du MCPBA ou de l'acide peracétique ou du perborate.

5. Procédé selon l'une des revendications 1 ou 2,
**caractérisé en ce que**
le précurseur fluoré au ¹⁸F est décarbonylé au moyen d'un catalyseur pour la production du composé de formule (2).

6. Procédé selon l'une des revendications 1 à 5,
**caractérisé en ce que**
l'ester ou le produit de décarbonylation est hydrolysé.

7. Procédé selon l'une des revendications 1 à 5,
**caractérisé en ce que**
le produit de formule (1) ou (2) est séparé par HPLC.
